# EUROPEAN PATENT SPECIFICATION

(11) **EP 0 577 204 B1**
(45) Date of publication and mention of the grant of the patent: **09.10.1996**
(21) Application number: 93201842.7
(22) Date of filing: 25.06.1993
(51) Int. Cl.: C07C 67/38, C07C 69/44, C07C 69/34

(54) **Preparation of alkanedioic derivatives**
Herstellung von Alkandicarbonsäurederivaten
Préparation de dérivés alcanedioiques

(30) Priority: 29.06.1992 EP 92201942
(43) Date of publication of application: 05.01.1994
(73) Proprietor: SHELL INTERNATIONALE RESEARCH MAATSCHAPPIJ B.V., 2596 HR Den Haag (NL)
(72) Inventor: Drent, Eit, NL-1031 CM Amsterdam (NL)

(56) References cited:
- EP-A- 0 198 521
- EP-A- 0 273 489

## Description

This invention relates to a process for the preparation of alkanedioic derivatives by reaction of an aliphatic conjugated diene with carbon monoxide and a hydroxyl group-containing compound in the presence of a palladium catalyst.

It is known that conjugated dienes can be carbonylated using various palladium catalysts. Depending on the nature of the hydroxyl group-containing compound used as coreactant, carboxylic esters will be obtained in the presence of alcohols, carboxylic acids in the presence of water, and carboxylic anhydrides in the presence of carboxylic acids.

Whilst dienes have two double bonds potentially available for carbonylation, most of the known processes result in the carbonylation of one of these double bonds only, and so in the formation of monofunctional alkenoic derivatives. Should the production of difunctional derivatives be desired, it is required to isolate this alkenoic intermediate product and to subject it to carbonylation of its remaining double bond in a separate step.

Thus, EP-A-284170 discloses a process for the preparation of adipic acid or esters, wherein 1,3-butadiene is carbonylated using a catalyst system comprising a palladium compound and a bidentate diphosphine ligand having aromatic substituents on the phosphorus atoms in a first step with formation of a pentenoic acid or ester, subsequent isolation of the pentenoic acid or ester obtained, followed by carbonylation of the isolated pentenoic acid or ester in the presence of a second carbonylation catalyst. The requirement of isolation of the intermediate product renders this known process more laborious.

EP-A-198521 discloses a process for the direct conversion of conjugated diene into carboxylic diacids or diesters, wherein use is made of a catalyst system comprising a palladium compound, an aryl-substituted monophosphine, optionally in conjunction with an aryl-substituted bidentate diphosphine, and at least one mol of hydrogen chloride per atom of trivalent phosphorus present in the catalytic system. Due to the corrosive nature of hydrogen chloride, this known process suffers from severe equipment requirements when being conducted on an industrial scale. Also, this known catalyst system leaves room for improvement regarding stability, as the monophosphine component tends to become inactivated under the conditions applied.

It has now been found that alkanedioic derivatives can directly be obtained from readily available diene feedstock under mild conditions using a non-corrosive catalyst system.

Accordingly, the present invention provides a process as indicated above, which is characterised in that use is made of a catalytic system comprising:
a) a source of cationic palladium,
b) a first bidentate diphosphine ligand having electron-withdrawing substituents on the phosphorus atoms,
c) a second bidentate diphosphine ligand having electron-releasing substituents on the phosphorus atoms, and
d) a source of an anion.

This process is advantageous in offering a direct route to alkanedioic derivatives, such as adipate esters, as shown in the below examples, as well as using a catalyst system of improved stability.

As used herein, the term "conjugated diene" refers to dienes having at least two double bonds which alternate with single bonds such as, for example, 1,3-butadiene, 1,3-pentadiene, 1,3-hexadiene, 1,3-cyclohexadiene, 2,4-heptadiene, or 2-methyl-1,3-butadiene. By being aliphatic "conjugated dienes", multienes having aromatically delocalised double bonds are excluded from the scope of the present invention. However, the aliphatic conjugated dienes may have non-aliphatic groups, such as phenyl groups, substituted onto the -C=C-C=C- backbone.

The hydroxyl group-containing compound used in the present process may be an alcohol, or a carboxylic acid or water. Accordingly, alkanedioic diesters, dianhydrides and diacids may be obtained by the present process. Preferably, the hydroxyl group-containing compound is an alcohol, more preferably an alkanol, and most preferably an alkanol having from 1 to 6 carbon atoms. Typical examples of suitable alcohols include methanol, ethanol, n-propanol, isopropanol, butanols. Also suitable are polyhydric alcohols such as ethylene glycol and 1,3-propanediol, which result in polyesters being produced.

The palladium catalyst used in the process of the invention may be provided in the form of a palladium complex of one or both of the specified diphosphines. It may also conveniently be generated in situ by adding a source of palladium and sources of the diphosphines to the reaction. Suitable sources of palladium include palladium carboxylates, such as palladium acetate, propionate, butyrate or benzoate, and palladium salts of mineral acids. Further sources include palladium complexes such as palladium acetylacetonate, tetrakis(triphenylphosphine)palladium and bis(tri-o-tolylphosphine)palladium acetate. Preferably the source of palladium is free of halide.

Palladium may be used in a heterogenised form, for example loaded on an ion exchange resin.

It is a characteristic feature of the present invention, that the catalyst system comprises a combination of at least two bidentate diphosphine ligands, which are distinguished by having electron withdrawing and electron releasing substitutents on the phosphorus atoms, respectively.

Preferably, the first bidentate diphosphine ligand comprises aromatic substituents. More preferably, the first bidentate diphosphine ligand is a diphosphine of formula:

R¹R²>P-R-P<R³R⁴ (I),

wherein R¹, R², R³ and R⁴ independently represent optionally substituted aryl groups, and R represents a bridging group having at least two carbon atoms in the bridge. Typical aryl groups represented by R¹, R², R³ and R⁴ include phenyl, naphthyl, o-methoxyphenyl, p-tolyl, o-tolyl, m-chlorophenyl and p-chlorophenyl.

Preferably, the second bidentate diphosphine ligand comprises aliphatic substituents. More preferably, the second bidentate diphosphine ligand is a diphosphine of formula:

R⁵R⁶>P-R-P<R⁷R⁸ (II),

wherein R⁵, R⁶, R⁷ and R⁸ independently represent optionally substituted aliphatic groups, and R represents a bridging group having at least two carbon atoms in the bridge. The optionally substituted aliphatic groups may be monovalent or divalent, in the latter case being bonded to a single or to both phosphorus atoms of the diphosphine ligand. Suitable aliphatic groups particularly include unsubstituted optionally branched or cyclic alkyl or alkylene groups having from 1 to 10 carbon atoms, more preferably from 1 to 6 carbon atoms. Preferably, R⁵, R⁶, R⁷ and R⁸ independently are selected from the group of alkyl, alkylene, cycloalkyl and cycloalkylene groups. Typical examples of aliphatic groups R⁵, R⁶, R⁷ and R⁸ include methyl, ethyl, propyl, isopropyl, n-butyl, s-butyl, t-butyl, cyclohexyl, pentamethylene, hexamethylene and cyclooctylene.

For being capable of bidentate coordination to the palladium atom, the bidentate diphosphine ligands of the catalyst system are inherently free of substituents offering steric hindrance to a bidentate coordination mode. In particular, the divalent bridging group R having at least two carbon atoms in the bridge, should be free of substituents offering steric hindrance, but otherwise can be any divalent group having two or more carbon atoms and optionally further heteroatoms, such as oxygen or nitrogen, in the bridge interconnecting both phosphorus atoms, and any further groups or atoms attached thereto. The bridging group R may make part of a cyclic structure, e.g. an aromatic or cycloaliphatic group, and the bonds in the bridge may be saturated or unsaturated. Also a 3-oxapentamethylene groups is suitable. Preferably, the bridging group R is an optionally substituted alkylene group having at least three carbon atoms in the chain, more preferably three or four carbon atoms. The first bidentate diphosphine ligand most preferably has four carbon atoms in the bridge, and the second ligand most preferably has three carbon atoms in the bridge.

Representative first bidentate diphosphine ligands include
1,3-bis(diphenylphosphino)propane, 1,4-bis(diphenylphosphino)butane, 1,3-bis(di-o-methoxyphenylphosphino)propane, 1,4-bis(di-p-chlorophenylphosphino)butane, and 1,4-bis(di-p-tolylphosphino)butane.

Representative second bidentate diphosphine ligands include
1,2-bis(di-n-butylphosphino)ethane, 1,3-bis(dimethylphosphino)propane, 1,3-bis(diethylphosphino)propane, 1,3-bis(di-i-propylphosphino)propane, 1,3-bis(di-n-propylphosphino)propane, 1,3-bis(di-i-butylphosphino)propane, 1,3-bis(di-n-butylphosphino)propane, 1,3-bis(di-s-butylphosphino)propane, 1,3-bis(di-t-butylphosphino)propane, 1,3-bis(di-n-hexylphosphino)propane, 1,2-bis(dicyclohexylphosphino)ethane, 1,3-bis(n-butylmethylphosphino)propane, 1,3-bis(n-butylethylphosphino)propane, 1,3-bis(1,5-cyclooctylenephosphino)propane and its isomeric mixture containing 1,4-cyclooctylene groups, 1,4-bis(di-i-propylphosphino)butane, 1,5-bis(dimethylphosphino)-3-oxapentane, 1,8-bis(di-n-butylphosphino)-3,6-dioxaoctane, and 1,4-bis(di-n-butylphosphino)-2,2,3,3-tetramethylbutane.

The ratio of the number of moles of either of the diphosphines per gram atom of palladium is preferably in the range of from 0.50 to 10, more preferably from 0.9 to 5, especially from 1 to 4. Preferably, the first bidentate diphosphine is used in molar excess relative to the second bidentate diphosphine, a ratio in the range of from 1:1 to 10:1 being most preferred.

The source of an anion, used in the process of the invention is preferably a protonic acid. However, it may also be a salt of palladium. It may also be a salt of another metal, for example of vanadium, chromium, nickel, copper or silver, or the salt of a protonated base when the protonic acid is neutralised with a base such as in 3,4-lutidinium salts. Preferably, the anion is derived from a weak protonic acid, in particular a carboxylic acid, having a pKa greater than 3, measured at 18 °C in aqueous solution, and being sterically hindered. Typically sterically hindered carboxylic acids, from which suitable anions may be derived, include 2,4,6-trimethylbenzoic acid, 2,6-dichlorobenzoic acid, 9-anthroic acid, pivalic acid, 1,2,3-benzenetricarboxylic acid and its 1,3-diester which may be formed in situ, 2-ethoxy-1-naphthalene carboxylic acid, and 2,6-dimethoxybenzoic acid.

The ratio of moles of anions per gram atom of palladium is not critical. Preferably it is in the range of from 0.5 to 100, more preferably in the range of from 1 to 10.

The diphosphines of formula I as such are known compounds, and can be prepared by general methods described in the literature, for example Houben-Weyl, Vol. XII/I, p.21.

The catalyst system used in the process according to the invention may be homogeneous or heterogeneous. Preferably it is homogeneous, or an immobilised homogeneous catalyst system.

The catalyst system according to the invention is preferably constituted in a liquid phase. The liquid phase may conveniently be formed by one or more of the reactants with which the catalyst system is to be used. Alternatively, it may be formed by a solvent. It may also be formed by one of the components of the catalyst system. Any inert solvent can be used. Said solvent may, for example, comprise sulphoxides and sulphones, for example dimethylsulphoxide, diisopropylsulphone or tetrahydrothiophene-2,2-dioxide (also referred to as sulfolane), 2-methylsulfolane, 3-methylsulfolane, 2-methyl-4-butylsulfolane; aromatic hydrocarbons such as benzene, toluene, and xylenes; esters such as methyl acetate and butyrolactone; ketones such as acetone or methyl isobutyl ketone; alcohols such as methanol and ethanol, ethers such as tetrahydrofurane (also referred to as THF), anisole, 2,5,8-trioxanonane (also referred to as diglyme), diphenyl ether and diisopropylether; and amides such as dimethylacetamide and N-methylpyrrolidone. Alcohols may function as coreactant to form esters.

The process according to the invention is conveniently effected at a temperature in the range of from 50 °C to 200 °C, in particular from 100 °C to 150 °C. Higher or lower temperature are not excluded, but usually don't provide any economic advantage.

The process according to the invention is preferably effected at a total pressure of from 1 to 80 bar. Pressures higher than 100 bar may be used, but are generally economically unattractive on account of special apparatus requirements. More preferred pressures

are in the range of from 5 to 70 bar. The process according to the invention may be carried out continuously or batchwise. Carbon monoxide grade, reaction equipment and product purification are not critical, and well within the skills of the relevant technician.

The invention will be illustrated in further detail by the following non-limiting examples.

### Example 1

A 300 ml magnetically stirred stainless-steel autoclave was charged with 20 ml ethanol, 40 ml diphenylether, 0.5 mmol palladium acetate, 2 mmol 1,4-bis(diphenylphosphino)butane, 0.6 mmol 1,3-bis(di-i-propylphosphino)propane and 10 mmol 9-anthracene carboxylic acid. The autoclave was flushed and evacuated, whereupon 10 ml of liquid 1,3-butadiene was added, and carbon monoxide introduced to an initial pressure of 40 bar. The autoclave was heated to 150 °C for 15 hours. Upon cooling, the contents of the autoclave were analysed by gas liquid chromatography (GLC). It was found that 100 % of the 1,3-butadiene was converted with a selectivity of 37 % into diethyl diesters of C⁶-alkanedioic acids and a selectivity of 60 % into ethyl monoesters of C⁵-alkenoic acids, with the diethyl C⁶-alkanedioates showing a linearity of 61 %, it means consisting for 61 % of diethyl adipate.

### Examples 2-6 and Comparative Example A

In the same way as Example 1, further experiments were conducted using the alcohol and catalyst components and their amounts as indicated in the below Table. After heating for 15 hours at the indicated temperatures and GLC analysis, the 1,3-butadiene conversions, the selectivities to diesters, the diester linearity and the selectivities to monoesters mentioned in the Table were observed. The following abbreviations are used in the Table:
- BDPbut =: 1,4-bis(diphenylphosphino)butane;
- BDiPP =: 1,3-bis(di-i-propylphosphino)propane;
- BcOP =: 1,3-bis(c-octylenephosphino)propane (isomeric mixture comprising 1,4- and 1,5-cyclooctylene groups);
- BDsBP =: 1,3-bis(di-s-butylphosphino)propane;
- BDcHP =: 1,3-bis(dicyclohexylphosphino)propane;
- 9-anth. =: 9-anthroic acid; DMBZ - 2,6-dimethoxybenzoic acid; EtOH - ethanol; MeOH - methanol.

**TABLE**

| Ex. No. | alcohol | 1° ligand | 2° ligand | anion | T | conv. | diester | | monoester |
|---|---|---|---|---|---|---|---|---|---|
| | (ml) | (mmol) | (mmol) | (mmol) | °C | % | sel. % | lin. % | sel. % |
| | | | | | | | | | |
| 1 | EtOH | BDPbut | BDiPP | 9-anth. | 150 | 100 | 37 | 61 | 60 |
| | (20) | (2.0) | (0.6) | (10) | | | | | |
| 2 | EtOH | BDPbut | BcOP | 9-anth. | 160 | 100 | 80 | 42 | 17 |
| | (20) | (2.0) | (0.6) | (10) | | | | | |
| 3 | EtOH | BDPbut | BDsBP | 9-anth. | 160 | 100 | 42 | 62 | 55 |
| | (20) | (2.0) | (0.6) | (10) | | | | | |
| 4 | EtOH | BDPbut | BDcHP | 9-anth. | 160 | 100 | 46 | 61 | 50 |
| | (20) | (2.0) | (0.6) | (10) | | | | | |
| 5 | EtOH | BDPbut | BDsBP | DMBZ | 160 | 100 | 34 | 60 | 64 |
| | (20) | (2.0) | (0.6) | (15) | | | | | |
| 6 | MeOH | BDPbut | BDiPP | 9-anth. | 160 | 100 | 30 | 67 | 66 |
| | (15) | (2.0) | (0.6) | (10) | | | | | |
| A | EtOH | BDPbut | - | 9-anth. | 150 | 100 | trace | | 97 |
| | (20) | (2.0) | | (10) | | | | | |

## Claims

1. A process for the preparation of alkanedioic derivatives by reaction of an aliphatic conjugated diene with carbon monoxide and a hydroxyl group-containing compound in the presence of a palladium catalyst, wherein the catalyst system comprises:
a) a source of cationic palladium,
b) a first bidentate diphosphine ligand having electron-withdrawing substituents on the phosphorus atoms,
c) a second bidentate diphosphine ligand having electron-releasing substituents on the phosphorus atoms, and
d) a source of an anion.

2. A process as claimed in claim 1, wherein the first bidentate diphosphine ligand comprises aromatic substituents.

3. A process as claimed in claim 2, wherein the first bidentate diphosphine ligand is a diphosphine of formula:
R¹R²>P-R-P<R³R⁴ (I),
wherein R¹, R², R³ and R⁴ independently represent optionally substituted aryl groups, and R represents a bridging group having at least two carbon atoms in the bridge.

4. A process as claimed in claim 1, wherein the second bidentate diphosphine ligand comprises aliphatic substituents.

5. A process as claimed in claim 4, wherein the second bidentate diphosphine ligand is a diphosphine of formula:
R⁵R⁶>P-R-P<R⁷R⁸ (II),
wherein R⁵, R⁶, R⁷ and R⁸ independently represent optionally substituted aliphatic groups, and R represents a bridging group having at least two carbon atoms in the bridge.

6. A process as claimed in claim 5, wherein R⁵, R⁶, R⁷ and R⁸ independently are selected from the group of alkyl, alkylene, cycloalkyl and cycloalkylene groups.

7. A process as claimed in any one or more of claims 1-6, wherein the aliphatic conjugated diene is 1,3-butadiene.

8. A process as claimed in any one or more of claims 1-7, wherein the hydroxyl group-containing compound is an alkanol having from 1 to 6 carbon atoms.

9. A process as claimed in any one or more of claims 1-8, wherein the first bidentate diphosphine ligand is used in molar excess relative to the second bidentate diphosphine ligand.

## Patentansprüche

1. Verfahren zur Herstellung von Alkandicarbonsäurederivaten durch Umsetzung eines aliphatischen konjugierten Diens mit Kohlenmonoxid und einer hydroxylgruppenhaltigen Verbindung in Gegenwart eines Palladiumkatalysators, wobei das Katalysatorsystem enthält:
a) eine Quelle für kationisches Palladium,
b) einen ersten zweizähnigen Diphosphinliganden mit elektronenanziehenden Substituenten an den Phosphoratomen,
c) einen zweiten zweizähnigen Diphosphinliganden mit elektronenabgebenden Substituenten an den Phosphoratomen und
d) eine Anionenquelle.

2. Verfahren nach Anspruch 1, wobei der erste zweizähnige Diphosphinligand aromatische Substituenten enthält.

3. Verfahren nach Anspruch 2, wobei es sich bei dem ersten zweizähnigen Diphosphinliganden um ein Diphosphin der Formel
R¹R²>P-R-P<R³R⁴ (I),
worin R¹, R², R³ und R⁴ unabhängig voneinander gegebenenfalls substituierte Arylgruppen bedeuten und R für eine Brückengruppe mit mindestens zwei Kohlenstoffatomen in der Brücke steht, handelt.

4. Verfahren nach Anspruch 1, wobei der zweite zweizähnige Diphosphinligand aliphatische Substituenten enthält.

5. Verfahren nach Anspruch 4, wobei es sich bei dem zweiten zweizähnigen Diphosphinliganden um ein Diphosphin der Formel
R⁵R⁶>P-R-P<R⁷R⁸ (II),
worin R⁵, R⁶, R⁷ und R⁸ unabhängig voneinander gegebenenfalls substituierte aliphatische Gruppen bedeuten und R für eine Brückengruppe mit mindestens zwei Kohlenstoffatomen in der Brücke steht, handelt.

6. Verfahren nach Anspruch 5, wobei R⁵, R⁶, R⁷ und R⁸ unabhängig voneinander aus der Gruppe Alkyl-, Alkylen-, cycloalkyl- und Cycloalkylengruppen ausgewählt sind.

7. Verfahren nach einem oder mehreren der Ansprüche 1-6, wobei es sich bei dem aliphatischen konjugierten Dien um 1,3-Butadien handelt.

8. Verfahren nach einem oder mehreren der Ansprüche 1-7, wobei es sich bei der hydroxylgruppenhaltigen Verbindung um ein Alkanol mit 1 bis 6 Kohlenstoffatomen handelt.

9. Verfahren nach einem oder mehreren der Ansprüche 1-8, wobei man den ersten zweizähnigen Diphosphinliganden im Verhältnis zum zweiten zweizähnigen Diphosphinliganden in molarem Überschuß verwendet.

## Revendications

1. Procédé de préparation de dérivés alcanedioïques par la réaction d'un diène conjugué aliphatique avec le monoxyde de carbone et un composé contenant un groupe hydroxyle, en présence d'un catalyseur au palladium, où le système catalytique comprend :
a) une source de palladium cationique,
b) un premier ligand de diphosphine bidentate possédant des substituants soutirant des électrons sur les atomes de phosphore,
c) un second ligand de diphosphine bidentate possédant des substituants libérant des électrons sur les atomes de phosphore et
d) une source d'un anion.

2. Procédé suivant la revendication 1, caractérisé en ce que le premier ligand de diphosphine bidentate comprend des substituants aromatiques.

3. Procédé suivant la revendication 2, caractérisé en ce que le premier ligand de diphosphine bidentate est une diphosphine de la formule :
R¹R²>P-R-P<R³R⁴ (I),
dans laquelle R¹, R², R³ et R⁴ représentent, chacun indépendamment, un radical aryle éventuellement substitué et R représente un groupe de pontage comportant au moins deux atomes de carbone dans le pont.

4. Procédé suivant la revendication 1, caractérisé en ce que le second ligand de diphosphine bidentate comprend des substituants aliphatiques.

5. Procédé suivant la revendication 4, caractérisé en ce que le second ligand de diphosphine bidentate est une diphosphine de la formule :
R⁵R⁶>P-R-P<R⁷R⁸ (II),
dans laquelle R⁵, R⁶, R⁷ et R⁸ représentent, chacun indépendamment, un radical aliphatique éventuellement substitué et R représente un groupe de pontage comportant au moins deux atomes de carbone dans le pont.

6. Procédé suivant la revendication 5, caractérisé en ce que R⁵, R⁶, R⁷ et R⁸ représentent, chacun indépendamment, un groupe alkyle, alkylène, cycloalkyle ou cycloalkylène.

7. Procédé suivant l'une quelconque ou plusieurs des revendications 1 à 6, caractérisé en ce que le diène conjugué aliphatique est le 1,3-butadiène.

8. Procédé suivant l'une quelconque ou plusieurs des revendications 1 à 7, caractérisé en ce que le composé contenant un groupe hydroxyle est un alcanol comportant de 1 à 6 atomes de carbone.

9. Procédé suivant l'une quelconque ou plusieurs des revendications 1 à 8, caractérisé en ce que le premier ligand de diphosphine bidentate est utilisé en un excès molaire par rapport au second ligand de diphosphine bidentate.
